# EUROPEAN PATENT APPLICATION

(11) **EP 3 290 400 A1**
(43) Date of publication of application: **07.03.2018**
(21) Application number: 17762037.4
(22) Date of filing: 23.03.2017
(51) Int. Cl.: C07C 41/58, C07C 43/317

(54) **METHOD FOR PRESERVATION OF , -DIFLUOROACETALDEHYDE ALKYL HEMIACETAL**

(30) Priority: 02.02.2017 JP 2017017983
(71) Applicant: CENTRAL GLASS COMPANY, LIMITED, Ube-shi, Yamaguchi 755-0001 (JP)
(72) Inventor: KIMURA, Masato, Ube-Shi, Yamaguchi 755-0001 (JP); AKIBA, Shinya, Kawagoe-shi, Saitama 350-1159 (JP); TAKEDA, Masaaki, Kawagoe-shi, Saitama 350-1159 (JP); TANAKA, Kazuki, Ube-Shi, Yamaguchi 755-0001 (JP); FUJIMOTO, Masataka, Ube-Shi, Yamaguchi 755-0001 (JP)
(74) Representative: Manitz Finsterwald Patentanwälte PartmbB
(86) International application number: PCT/JP2017/011564
(87) International publication number: WO 2018/016126

(57) **Abstract**

Disclosed is a method for preserving an α,α-difluoroacetaldehyde alkyl hemiacetal of the following formula in a gas-liquid state having gas and liquid phases in a closed container under an atmosphere of oxygen (O₂) or inert gas, characterized by: controlling the oxygen concentration of the gas phase in the container to be 5000 ppm or lower; and then storing the α,α-difluoroacetaldehyde alkyl hemiacetal in the container under light-shielding conditions 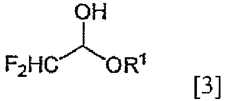 where R¹ represents an alkyl group or a substituted alkyl group.

It is possible by this method to suppress conversion of the α,α-difluoroacetaldehyde alkyl hemiacetal to difluoroacetic acid over a long term.

## Description

### Field of the Invention

The present invention relates to a method for preserving an α,α-difluoroacetaldehyde alkyl hemiacetal.

### Background Art

It is known that α,α-difluoroacetaldehyde, which is represented by the formula (1), is a compound useful as raw materials for advanced materials or intermediates for pharmaceutical and agrichemical products.

CHF₂-CHO [1]

In particular, α,α-difluoroacetaldehyde has a difluoromethyl group (-CHF₂) in which two fluorine atoms of high electronegativity and one hydrogen atom are bonded to the same carbon atom. It is considered that this specific structure is deeply relevant to the properties of various materials produced therewith, such as water repellency, transparency, low dielectric constant, peculiar physiological activity and mimic effect. Consequently, materials produced using α,α-difluoroacetaldehyde as building blocks are becoming subjects of vigorous researches and developments in the fields of advanced materials and pharmaceutical and agrichemical intermediates.

There has conventionally been known a method of synthesizing α,α-difluoroacetaldehyde by reduction of a difluoromethyl-containing ester with a hydride reduction agent such as lithium aluminum hydride in the presence of a catalyst (see Non-Patent Document 1). Further, the present applicant has disclosed a method of synthesizing α,α-difluoroacetaldehyde by reduction of an α,α-difluoroacetate with hydrogen (H₂) in the presence of a ruthenium catalyst (see Patent Document 1).

On the other hand, it is known that an aldehyde is unstable and gradually polymerized with another aldehyde molecule (see Non-Patent Document 2). It is also described in Patent Document 1 that the target aldehyde compound of the present invention is obtained as a plurality of stable equivalents, such as self-polymerization product, hydrate, hemiacetal, acetal, and compound with structural features thereof, because of the direct bonding of a strong electron-withdrawing difluoromethyl group to the aldehyde carbon.

As mentioned above, the aldehyde in which the difluoromethyl group is directly bonded to the aldehyde carbon tends to be easily converted to a plurality of compounds. The present applicant has found a unique phenomenon that, when α,α-difluoroacetaldehyde is converted to an α,α-difluoroacetaldehyde alkyl hemiacetal of the general formula [3] in the coexistence of an alcohol of the general formula [2], the hemiacetal is likely to exist stably in the system and significantly less likely to be converted to a compound other than the hemiacetal (such as dimer) by controlling the amount of the alcohol coexisting in the system as reported in Patent Document 2.

R¹-OH [2]

In the general formula [2], R¹ represents an alkyl group or a substituted alkyl group. 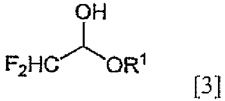 In the general formula [3], R¹ has the same meaning as in the general formula [2].

### Prior Art Documents

### Patent Documents

Patent Document 1: International Publication No. 2014/115801
Patent Document 2: International Publication No. 2016/017318

### Non-Patent Documents

Non-Patent Document 1: Journal of Organic Chemistry, 1997, 62(25), 8826-8834
Non-Patent Document 2: Synthetic Organic Chemistry, vol. 19, no. 3 (1961), p. 254-260

### Summary of the Invention

### Problems to be Solved by the Invention

Since it is a known fact that α,α-difluoroacetaldehyde is obtained as stable equivalents such as self-polymerization product, hydrate, hemiacetal etc. as discussed in the above-mentioned patent documents, a person skilled in the art could easily form an α,α-difluoroacetaldehyde alkyl hemiacetal of the general formula [3] by coexistence of α,α-difluoroacetaldehyde and alcohol with reference to these patent documents.

Further, the method of Patent Document 2 enables long-term preservation of a hemiacetal of α,α-difluoroacetaldehyde and suppresses formation of an α,α-difluoroacetaldehyde dimer of the general formula [4] (hereinafter simply referred to as "dimer" in the present specification), which is a phenomenon peculiar to this hemiacetal compound. 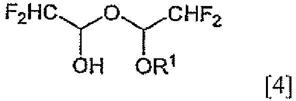 In the general formula [4], R¹ has the same meaning as in the general formula [2].

The method of Patent Document 2 is effective for preservation of the hemiacetal. However, the method of Patent Document 2 has several limitations on the preservation conditions (i.e. needs to control the pH of the system to be neutral, control the water content of the system to be 1000 ppm or lower and control the total molar amount of the alcohol to be 1.15 to 4.00 times the total molar amount of the α,α-difluoroacetaldehyde in the system) and cannot always be said as an easy method for long-term preservation of the hemiacetal.

Even though the preservation stability of the α,α-difluoroacetaldehyde can be improved by conversion to the hemiacetal as mentioned above, the resulting difluoroacetaldehyde hemiacetal may undergo decomposition to generate difluoroacetic acid as a by-product during the preservation (see the after-mentioned comparative examples). The difluoroacetic acid itself is a strongly acidic compound. The material of the reaction container is affected by the difluoroacetic acid as the amount of the difluoroacetic acid generated is increased. The generation of the difluoroacetic acid is hence not suitable for long-term preservation of the α,α-difluoroacetaldehyde.

There has thus been a demand to find out the conditions for preventing decomposition of the difluoroacetaldehyde hemiacetal during the preservation, i.e., found out techniques for improving the preservation stability of the difluoroacetaldehyde hemiacetal.

### Means for Solving the Problems

In view of the foregoing problems, the present inventors have made extensive researches and resultantly found that, when an α,α-difluoroacetaldehyde alkyl hemiacetal of the general formula [3] is preserved in a gas-liquid state having gas and liquid phases in a closed container under an inert gas atmosphere, it is possible to suppress generation of difluoroacetic acid without causing decomposition of the hemiacetal by controlling the oxygen concentration of the gas phase to be 5000 ppm or lower and shielding the hemiacetal under light-shielding conditions. The present invention is based on such a finding.

Namely, the present invention includes the following inventive aspects 1 to 6.

### [Inventive Aspect 1]

A method for preserving an α,α-difluoroacetaldehyde alkyl hemiacetal of the general formula [3] in a gas-liquid state having gas and liquid phases in a closed container under an atmosphere of oxygen (O₂) or inert gas, the method comprising:
controlling the oxygen (O₂) concentration of the gas phase in the container to be 5000 ppm or lower; and then
storing the α,α-difluoroacetaldehyde alkyl hemiacetal in the container under light-shielding conditions.

### [Inventive Aspect 2]

The method according to Inventive Aspect 1, wherein the storing is carried out in a temperature range of -50°C to 80°C.

### [Inventive Aspect 3]

A method for preserving an α,α-difluoroacetaldehyde alkyl hemiacetal, comprising the following steps:
[First Step] feeding an α,α-difluoroacetaldehyde alkyl hemiacetal of the general formula [3] into a container, thereby forming a gas-liquid state having a gas phase and a liquid phase of the α,α-difluoroacetaldehyde alkyl hemiacetal in the container;
[Second Step] after the first step, controlling the oxygen concentration of the gas phase in the container to be 5000 ppm or lower by charging oxygen (O₂) or inert gas into the container; and
[Third Step] after the second step, closing the container and then storing the α,α-difluoroacetaldehyde alkyl hemiacetal in the container under light-shielding conditions.

### [Inventive Aspect 4]

The method according to Inventive Aspect 2, wherein, in the third step, the storing is carried out in a temperature range of -50°C to 80°C.

### [Inventive Aspect 5]

The method according to any one of Inventive Aspects 1 to 4, further comprising: producing the α,α-difluoroacetaldehyde alkyl hemiacetal of the general formula [3] by reaction of an α,α-difluoroacetate of the general formula [5] with hydrogen (H₂) in an alcohol solvent of the general formula [2] in the presence of a base and a ruthenium catalyst; and using the hemiacetal as a starting raw material 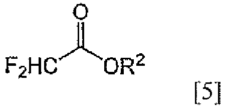 where R² represents an alkyl group or a substituted alkyl group.

### [Inventive Aspect 6]

The method according to any one of Inventive Aspects 1 to 5, wherein, in the α,α-difluoroacetaldehyde alkyl hemiacetal of the general formula [3], R¹ is a methyl group or ethyl group.

It is possible according to the present invention to suppress generation of difluoroacetic acid as a product of decomposition of the α,α-difluoroacetaldehyde alkyl hemiacetal.

### Detailed Description of the Embodiments

Hereinafter, the respective features of the present invention will be described below in detail. It should be understood that: the present invention is not limited to the following description; and various changes and modifications of the following embodiments can be made as appropriate without departing from the scope of the present invention. All of the publications cited in the present specification, such as prior art documents, unexamined patent publications, patent publications and other patent documents, are herein incorporated by reference.

In the target compound of the present invention, that is, the α,α-difluoroacetaldehyde alkyl hemiacetal of the general formula [3], R¹ is an alkyl group or a substituted alkyl group. The meaning of R¹ in the dimer of the general formula [4] is the same as that of R¹ in the hemiacetal.

The alkyl group preferably refers to a C₁-C₁₀ linear or branched alkyl group or a C₃-C₁₀ cyclic alkyl group. (In the present specification, the term "alkyl group" means an "unsubstituted alkyl group".) Specific examples of the alkyl group are methyl, ethyl, n-propyl, i-propyl, n-butyl, i-butyl, s-butyl, t-butyl, n-pentyl, n-octyl, n-decyl, cyclopropyl, cyclobutyl, cyclopentyl and cyclohexyl. The substituted alkyl group refers to a group obtained by substitution of any number of and any combination of substituents onto carbon atoms of the above alkyl group. As such substituents, there can be used halogen atoms, lower alkoxy groups, lower haloalkoxy groups, cyano group, lower alkoxycarbonyl groups and the like. Specific examples of the substituents are fluorine, chlorine, bromine, methoxy, ethoxy, propoxy, fluoromethoxy, chloromethoxy, bromomethoxy, methoxycarbonyl, ethoxycarbonyl and propoxycarbonyl. In the present specification, the term "lower" means that the group to which the term is attached has 1 to 6 carbon atoms in the form of a linear or branched chain structure or a cyclic structure (in the case of 3 carbons or more).

It is preferable to use a methyl group or ethyl group as R¹, that is, use α,α-difluoroacetaldehyde methyl hemiacetal or α,α-difluoroacetaldehyde ethyl hemiacetal as the α,α-difluoroacetaldehyde alkyl hemiacetal of the general formula [3] in view of ease of material availability.

The α,α-difluoroacetaldehyde alkyl hemiacetal of the general formula [3] to be preserved according to the present invention can be produced by reaction of an α,α-difluoroacetate of the general formula [5] with hydrogen in an alcohol of the general formula [2] as a solvent in the presence of a base and a ruthenium catalyst as disclosed in Patent Document 1.

Although the hemiacetal can alternatively be produced by other known methods such as hydride reduction of a difluoroacetate as disclosed in Non-Patent Document 1, the above-mentioned method of Patent Document 1 is particularly advantageous for mass production of the hemiacetal and is important for the present invention. The production of the hemiacetal by this method will be hence explained in detail below.

In the α,α-difluoroacetate of the general formula [5], R² is an alkyl group or a substituted alkyl group. The alkyl and substituted alkyl groups in the general formula [5] means the same as R¹ in the general formula [3] or general formula [4].

There is no particular limitation on the kind of the ruthenium catalyst used. For example, a ruthenium catalyst of the following general formula [6] or formula [7] is preferably used. 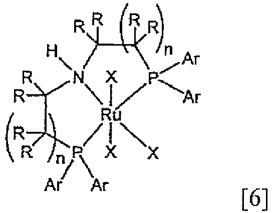 In the general formula [6], R each independently represents a hydrogen atom, an alkyl group, a substituted alkyl group, an aromatic ring group or a substituted aromatic ring group; Ar each independently represents an aromatic ring group or a substituted aromatic ring group; X each independently represents a ligand with a formal charge of -1 or 0 (with the proviso that the sum of the formal charges of three X is -2); and *n* each independently represent an integer of 1 or 2. 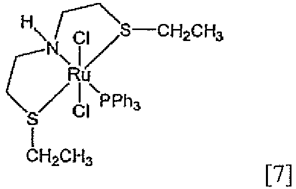 In the formula [7], Ph represents a phenyl group.

The alkyl group of the ruthenium catalyst of the general formula [6] means the same as R¹ in the general formula [3] or general formula [4]. The aromatic ring group of the ruthenium catalyst refers to an aromatic hydrocarbon group or an aromatic heterocyclic group containing a hetero atom e.g. nitrogen atom, oxygen atom or sulfur atom. Specific examples of the aromatic hydrocarbon group are C₆-C₁₈ aromatic hydrocarbon groups such as phenyl, naphthyl and anthryl. Specific examples of the aromatic heterocyclic group are pyrrolyl (including nitrogen protected form), pyridyl, furyl, thienyl, indolyl (including nitrogen protected form), quinolyl, benzofuryl and benzothienyl.

The substituted alkyl and aromatic ring groups of the ruthenium catalyst of the general formula [6] refer to those obtained by substitution of any number of and any combination of substituents onto carbon atoms of the above alkyl and aromatic ring groups. As such substituents, there can be used halogen atoms,lower alkyl groups, lower haloalkyl groups, lower alkoxy groups, lower haloalkoxy groups, cyano group, lower alkoxycarbonyl groups, aromatic ring groups, carboxyl group, protected carboxyl groups, amino group, protected amino groups, hydroxyl group and protected hydroxyl groups. Specific examples of these substituents are fluorine, chlorine, bromide, methyl, ethyl, propyl, fluoromethyl, chloromethyl, bromomethyl, methoxy, ethoxy, propoxy, fluoromethoxy, chloromethoxy, bromomethoxy, methoxycarbonyl, ethoxycarbonyl, propoxycarbonyl, phenyl, naphthyl, anthryl, pyrrolyl (including nitrogen protected form), pyridyl, furyl, thienyl, indolyl (including nitrogen protected form), quinolyl, benzofuryl and benzothienyl.

In the alkyl group of the ruthenium catalyst of the general formula [6], an arbitrary carbon-carbon single bond or bonds may be replaced by any number of and any combination of carbon-carbon double bonds and carbon-carbon triple bonds. (As a matter of course, the alkyl group with such an unsaturated bond or bonds may have any of the above substituents.) Depending on the kind of the substituent, the substituent itself may be involved in a side reaction. The side reaction can however be minimized by the adoption of suitable reaction conditions. The aromatic ring groups described above as "such substituents" may further be substituted with a halogen atom, lower alkyl group, lower haloalkyl group, lower alkoxy group, lower haloalkoxy group, cyano group, lower alkoxycarbonyl group, carboxyl group, protected carboxyl group, amino group, protected amino group, hydroxyl group, protected hydroxyl group etc. As the protecting groups of the pyrrolyl, indolyl, carboxyl, amino and hydroxyl groups, there can be used those described in "Protective Groups in Organic Synthesis", Third Edition, 1999, John Wiley & Sons, Inc.

Among the ruthenium catalyst of the general formula [6], a ruthenium catalyst of the following formula (commercially available under the trade name of "Ru-MACHO™" from Takasago International Corporation) is particularly preferred because of its high activity. 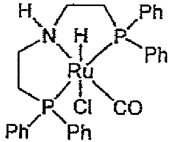 In the above formula, Ph represents a phenyl group.

Although the reaction of the difluoroacetate and hydrogen needs to be performed in the presence of the base, it is feasible to perform the reaction in the absence of the base in the case where at least one of three X ligands of the ruthenium catalyst is BH₄.

As the ruthenium catalyst of the formula [7], it is convenient to use a ruthenium catalyst commercially available under the trade name "Ru-SNS" from Sigma-Aldrich Co. LLC. although the ruthenium catalyst of the formula [7] can be prepared by a known method.

In place of the above-exemplifed ruthenium catalysts, there can be used any ruthenium catalysts described in: Angew. Chem. Int. Ed., 2013, 52, 2538-2542; Organometallics, 2012, 31, 5239-5242; Angew. Chem. Int. Ed., 2012, 51, 2772-2775; and Angew. Chem. Int. Ed., 2006, 45, 1113-1115. Typical examples of such ruthenium catalysts (homogeneous ruthenium catalysts) are indicated in FIG. 1 (where Et: ethyl, t-Bu: tert-butyl, Ph: phenyl, i-Pr: isopropyl). As a matter of course, the ruthenium catalyst is not limited to these examples. These ruthenium catalysts can be used under the same reaction conditions as the above-mentioned ruthenium catalysts.

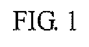

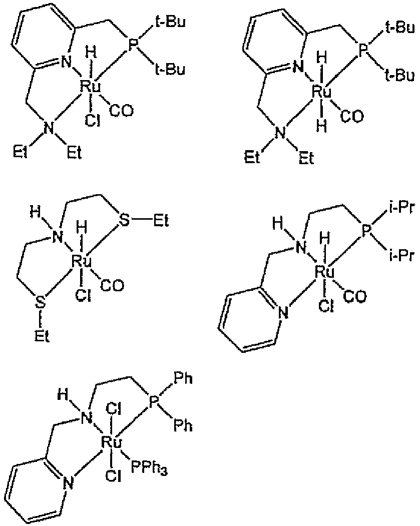

The amount of the ruthenium catalyst used is generally 0.000001 mol or more, preferably 0.00001 to 0.005 mol, more preferably 0.00002 to 0.002 mol, per 1 mol of the α,α-difluoroacetate.

As the base, there can be used alkali metal hydrogencarbonates, alkali metal carbonates, alkali metal hydroxides, tetraalkyl ammonium hydroxides, alkali metal alkoxides, organic bases, alkali metal bis(trialkylsilyl)amides and alkali metal borohydrides. Specific examples of the base are lithium hydrogencarbonate, sodium hydrogencarbonate, potassium hydrogencarbonate, lithium carbonate, sodium carbonate, potassium carbonate, lithium hydroxide, sodium hydroxide, potassium hydroxide, tetramethyl ammonium hydroxide, tetraethyl ammonium hydroxide, tetra-n-propyl ammonium hydroxide, tetra-n-butyl ammonium hydroxide, lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, triethylamine, diisopropylethylamine, 4-dimethylaminopyridine, 1,8-diazabicyclo[5.4.0]undec-7-ene, lithium bis(trialkylsilyl)amide, sodium bis(trialkylsilyl)amide, potassium bis(trialkylsilyl)amide, lithium borohydride, sodium borohydride and potassium borohydride. Among others, alkali metal alkoxides (whose carbon number is 1 to 6) are preferred. Particularly preferred are lithium methoxide, sodium methoxide and potassium methoxide.

In general, sodium methoxide is available in the form of a methanol solution as in the after-mentioned synthesis example. In the case of using such a methanol solution of sodium methoxide, methanol remains in the reaction system and acts as at least a part of the alcohol of the general formula [2].

The meaning of R¹ in the alcohol of the general formula [2] is the same as that of R¹ in the above α,α-difluoroacetaldehyde alkyl hemiacetal of the general formula [3].

Specific examples of the alcohol are methanol, ethanol, n-propanol, isopropanol, butanol, tert-butanol and benzyl alcohol. Among others, methanol, ethanol, n-propanol and isopropanol are preferred. Particularly preferred are methanol and ethanol, each of which is readily available as a dehydrated reagent on a large scale and has a large stabilization effect on the α,α-difluoroacetaldehyde alkyl hemiacetal.

In the case of using the base, the amount of the base used is generally 0.001 mol or more, preferably 0.005 to 5 mol, more preferably 0.01 to 3 mol, per 1 mol of the α,α-difluoroacetate used as the raw substrate material.

The amount of the hydrogen gas used is generally 1 mol or more per 1 mol of the α,α-difluoroacetate. Preferably, the hydrogen gas is used in a large excessive amount. It is particularly preferable to use the hydrogen gas in a largely excessive amount under pressurized conditions.

There is no particular limitation on the hydrogen gas pressure. The hydrogen gas pressure is generally 10 to 0.41 MPa (in terms of absolute pressure; the same applies to the following), preferably 6 to 0-1 MPa, more preferably 5 to 0.3 MPa.

The amount of the reaction solvent used is generally 0.03 L (liter) or more, preferably 0.05 to 10 L, more preferably 0.07 to 7 L, per 1 mol of the α,α-difluoroacetate used as the raw substrate material.

The reaction time is generally 72 hours or less. As the reaction time varies depending on the raw substrate material and reaction conditions, it is preferable to determine the time at which there is seen almost no decrease of the raw substrate material as the end of the reaction while monitoring the progress of the reaction by any analytical means such as gas chromatography, liquid chromatography or nuclear magnetic resonance.

As described above, the α,α-difluoroacetaldehyde alkyl hemiacetal of the general formula [3] can be produced and used as the starting raw material for the preservation method of the present invention.

In the above hydrogenation reaction, α,α-difluoroacetaldehyde (as corresponding to the general formula [1]) is once formed and then promptly converted to a stable alkyl hemiacetal (i.e. the α,α-difluoroacetaldehyde alkyl hemiacetal of the general formula [3]) by reaction the alcohol in the system. The starting raw material for the preservation method of the present invention may thus contain the alcohol of the general formula [2] and the dimer of the general formula [4] in addition to the α,α-difluoroacetaldehyde alkyl hemiacetal of the general formula [3]. The preservation method of the present invention is suitably applicable to such a starting raw material.

Next, the preservation method of the present invention will be explained below.

The preservation method of the present invention is for preserving the α,α-difluoroacetaldehyde alkyl hemiacetal of the general formula [3] in a gas-liquid state having gas and liquid phases in a closed container under an atmosphere of oxygen (O₂) or inert gas, characterized by: controlling the oxygen concentration of the gas phase in the container to be 5000 ppm or lower; and then storing the α,α-difluoroacetaldehyde alkyl hemiacetal in the container under light-shielding conditions.

The present invention includes, as one embodiment, the case where the preservation method takes place through the following steps. The respective steps will be successively explained below.
[First Step] The α,α-difluoroacetaldehyde alkyl hemiacetal of the general formula [3] is fed into the container, thereby forming a gas-liquid state having a gas phase and a liquid phase of the α,α-difluoroacetaldehyde alkyl hemiacetal in the container;
[Second Step] After the first step, the container is closed; and the oxygen concentration of the gas phase in the container is controlled to be 5000 ppm or lower by charging oxygen (O₂) or inert gas into the container; and
[Third Step] After the second step, the container is closed; and then the α,α-difluoroacetaldehyde alkyl hemiacetal is stored in the container under light-shielding conditions.

### [First to Third Steps]

Any container such as glass container (including glass-lined container), stainless steel container etc. is usable as the container as long as the container is capable of, after the α,α-difluoroacetaldehyde alkyl hemiacetal is fed into the container, being closed to maintain therein the gas-liquid state and is made of a material that does not allow permeation of oxygen under light-shielding conditions. The container can be in the form of e.g. a fixed storage container such as storage tank or a transportable container such as drum.

More specifically, the α,α-difluoroacetaldehyde alkyl hemiacetal is first fed into the container. The amount of the α,α-difluoroacetaldehyde alkyl hemiacetal charged into the container is generally 20% or more and less than 99%, preferably 50% or more and less than 95%, of the volume of the container. Since the charge amount depends on the size of the container used, it is preferable to feed the hemiacetal into the container while adjusting the charge amount to within the above range. When the hemiacetal is charged in an amount less than 20% of the volume of the container, the amount of preservation of the hemiacetal is small. This leads to a large number of operations required for preservation in the actual production process of the hemiacetal. Such small-amount preservation cannot thus be said to be economical. When the hemiacetal is charged in an amount of 99% or more of the volume of the container, by contrast, there may occur breakage of the container or leakage of the liquid from inside of the container due to variations in the volume of the hemiacetal caused by variations in the temperature of the outside environment (i.e. the outside of the container). In order to maintain the oxygen concentration range in the closed container as disclosed by the present invention, it is necessary to consider not only the oxygen concentration of the gas phase but also the dissolved oxygen concentration of the liquid phase (for the reason that the oxygen concentration inside the container may vary with time and exert an influence on the preservation effects of the present invention).

After the hemiacetal is fed into the container, the inert gas such as nitrogen or argon is charged into the container. There is no particular limitation on the method for charging the inert gas into the container. It is for example feasible to, after feeding the α,α-difluoroacetaldehyde alkyl hemiacetal into the container, bubble the inert gas into the liquid phase (in which the α,α-difluoroacetaldehyde alkyl hemiacetal is contained) inside the container and then close the container, or close the container, vacuum the container to a degree that does not cause the α,α-difluoroacetaldehyde alkyl hemiacetal to be discharged out of the container and then blow or bubble the inert gas into the liquid phase inside the container, as in the after-mentioned working examples. In either case, the dissolved oxygen concentration of the liquid phase is decreased and, at the same time, the gas phase inside the container is gradually replaced with the inner gas.

In order to improve the dissolved oxygen removal efficiency, it is preferable to utilize stirring operation or degassing operation of the liquid phase in combination of the above charging method. The oxygen concentration can be efficiently decreased by appropriately combining these operations.

Any gas that does not affect the reaction, such as nitrogen, argon or the like, is usable as the inert gas.

Subsequently, the oxygen concentration inside the container is adjusted so that the oxygen concentration of the gas phase becomes 5000 ppm or lower, preferably 1000 ppm or lower, more preferably 300 ppm or lower, in the present invention. There is no particular limitation on the method for controlling the oxygen concentration. For example, the following methods can be used: (1) the oxygen concentration is controlled to within the above range by introducing the inert gas into the container; (2) the oxygen concentration inside the container is decreased to the appropriate range by blowing a mixed gas of the oxygen and the inert gas such as nitrogen or argon; and (3) the oxygen concentration is controlled by closing and vacuuming the container into which the α,α-difluoroacetaldehyde alkyl hemiacetal is charged. In the case of blowing the mixed gas of the oxygen and the inert gas such as nitrogen or argon, there is no particular limitation on the mixing ratio of the oxygen and the inert gas in the mixed gas.

By the adoption of these conditions, it is possible to sufficiently prevent reaction such as polymerization or oxidization of the α,α-difluoroacetaldehyde alkyl hemiacetal. The above control method (1) or (2) is preferably used because it is easier to control the oxygen concentration of the oxygen concentration to be 5000 ppm or lower.

It is conceivable to control the oxygen concentration of the gas phase to be lower than 10 ppm (e.g. 0 ppm to lower than 3 ppm). Since the preservation effects of the present invention can be sufficiently obtained within the above oxygen concentration range, however, there is no need to decrease the oxygen concentration to an extreme limit of e.g. about 0 ppm. In order to decrease the oxygen concentration to an extreme limit level, the container needs to be closed and vacuumed under extreme conditions after the charging of the α,α-difluoroacetaldehyde alkyl hemiacetal into the container. Under such extreme vacuum conditions, the α,α-difluoroacetaldehyde alkyl hemiacetal itself may be discharged out of the container. For these reasons, it is a preferable embodiment to control the oxygen concentration to within the range disclosed in the after-mentioned working examples in terms of less load on equipment and operation process.

There is no particular limitation on the storage temperature. In general, the hemiacetal is stored in a temperature range of -50°C to +80°C. The hemiacetal is preferably stored in the temperature range -40°C to +70°C, more preferably in the vicinity of room temperature (i.e. 10 to 30°C).

### Examples

The present invention will be described in more detail below by way of the following examples. It should however be understood that the following examples are not intended to limit the present invention thereto. Herein, the quantification results (composition ratio and yield) of each product was based on the composition ratio "mol%" of a reaction mixture as measured by means of a nuclear magnetic resonance (NMR) analyzer. Further, the term "n.d." in each table means that the product was not detected.

### [Synthesis Example] Synthesis of α,α-difluoroacetaldehyde ethyl hemiacetal (abbreviated as DFAL-EtOH)

Into a pressure-resistant reaction vessel of stainless steel, 109 g (0.88 mol) of ethyl α,α-difluoroacetate, 0.107 g (0.18 mmol) of the following ruthenium catalyst, 42 g of 28% sodium methoxide methanol solution (containing 0.22 mol of sodium methoxide) and 290 mL of methanol were put. The inside of the reaction vessel was replaced five times with hydrogen gas. The hydrogen pressure inside the reaction vessel was set to 1.0 MPa. Subsequently, the contents of the reaction vessel were reacted with stirring for 8 hours at 15°C.

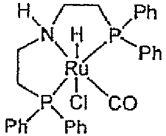

After the completion of the reaction, it was confirmed by ¹⁹F-NMR analysis that: the conversion rate of the ethyl α,α-difluoroacetate was 65%; and the selectivity of DFAL-EtOH was 91%. In the ¹⁹F-NMR analysis, quantification was performed using α,α,α-trifluorotoluene as internal standard substance.

With the addition of 13.2 g (0.22 mol) of acetic acid to the reaction completed solution, the reaction completed solution was changed to a pH of 8. Thus, the addition of the acetic acid was stopped upon judging that the pH of the reaction completed became substantially neutral. This solution was directly subjected to distillation (bottom temperature: ∼ 66°C, vacuum degree: ∼ 2.1 kPa), thereby obtaining a methanol solution containing DFAL-EtOH. The thus-obtained solution was subjected to precision distillation (theoretical plate number: 35, distillation temperature: 92°C, vacuum degree: ∼ 35 kPa) so as to separate therefrom a major portion of methanol. The distillation was continued after 96 g (2.08 mol) of ethanol was added to the distillation bottom. As a result, 45.9 g of DFAL-EtOH was yielded as a fraction.

It was confirmed that ethanol, DFAL-EtOH and ethyl hemiacetal dimer of the following formula were contained in the fraction. The purity (mol%) of the ethanol, DFAL-EtOH and the dimer were 8.6 wt%, 83.6 wt% and 7.1 wt%, respectively. In view of the purity, the yield was 41%.

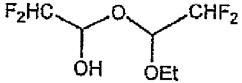

The following examples and comparative examples were carried out using the fraction obtained in the above synthesis example.

### [Examples 1 to 2 and Comparative Examples 1 to 2]

Into a glass container with an internal volume of 100 ml, 50 g of the mixed solution of α,α-difluoroacetaldehyde ethyl hemiacetal (83.6 wt%), ethanol (8.6 wt%) and the following dimer (7.1 wt%) was charged. After nitrogen was charged into the container, the mixed solution was subjected to bubbling treatment with nitrogen gas.

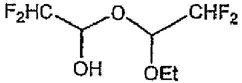

When the oxygen concentration inside the container was adjusted to about 16000 ppm (Example 1) or about 7000 ppm (Example 2) by the bubbling treatment, a mixed gas of oxygen and nitrogen was charged into the container so as to control the oxygen concentration inside the container to a value indicated in TABLE 1. After that, the container was light-shielded and sealed.

The sealed container was placed in a thermostat and left for 24 hours under constant-temperature conditions of 70°C. After the lapse of 24 hours, the container was taken out of the thermostat. The solution inside the container was tested for the presence of a decomposition product by means of a nuclear magnetic resonance (NMR) analyzer. The test results are shown in TABLE 1.

**TABLE 1**

| | Oxygen conc. (ppm) inside container | Presence of difluoroacetic acid | Amount (ppm) of difluoroacetic acid generated relative to hemiacetal |
|---|---|---|---|
| Example 1 | 2000 | not present | n.d. |
| Example 2 | 4000 | not present | n.d. |
| Comparative Example 1 | 6000 | present | 131 |
| Comparative Example 2 | 8000 | present | 86 |

The generation of difluoroacetic acid, which would become a problem in practical use, was not observed in Examples 1 to 2 as shown in TABLE 1. On the other hand, the generation of difluoroacetic acid as a decomposition product was observed in Comparative Examples 1 to 2.

### [Example 3 and Comparative Examples 3 to 5]

Into a quartz cell container with an internal volume of 10 ml, 5.0 g of the mixed solution of α,α-difluoroacetaldehyde ethyl hemiacetal (83.6 wt%), ethanol (8.6 wt%) and the following dimer (7.1 wt%) was charged under a nitrogen atmosphere. The mixed solution was subsequently subjected to bubbling treatment with nitrogen gas.

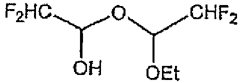

When the oxygen concentration inside the container was adjusted to 6000 ppm by the bubbling treatment, a mixed gas of oxygen and nitrogen was charged into the container so as to control the oxygen concentration inside the container to a value indicated in TABLE 2. After that, the container was sealed.

The container was left for 7 hours at 25°C under various light irradiation conditions. The solution inside the container was tested for the presence of a decomposition product. The test results are shown in TABLE 2.

**TABLE 2**

| | Oxygen conc. (ppm) inside container | Light irradiation conditions | Presence of difluoroacetic acid | Amount (ppm) of difluoroacetic acid generated relative to hemiacetal |
|---|---|---|---|---|
| Example 3 | 2000 | light shielding | not present | n.d. |
| Comparative Example 3 | 2000 | ultraviolet irradiation (135 nm) | present | 135 |
| Comparative Example 4 | 2000 | ultraviolet irradiation (245 nm) | present | 96 |
| Comparative Example 5 | 2000 | sunlight | present | 50 |

As shown in TABLE 2, the generation of difluoroacetic acid, which would become a problem in practical use, was not observed in Example 3. It is thus apparent in Example 3 that there occurred no decomposition of the α,α-difluoroacetaldehyde alkyl hemiacetal. On the other hand, the generation of difluoroacetic acid as a decomposition product was observed in Comparative Examples 3 to 5.

### [Examples 4 to 5 and Comparative Examples 6 to 10]

Into a transparent or light-shielded glass container with an internal volume of 100 ml, 50 g of the mixed solution of α,α-difluoroacetaldehyde ethyl hemiacetal (83.6 wt%), ethanol (8.6 wt%) and the following dimer (7.1 wt%) was charged under a nitrogen atmosphere. The mixed solution was subsequently subjected to bubbling treatment with nitrogen gas.

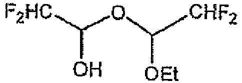

By the bubbling treatment, the oxygen concentration inside the container was controlled to a value indicated in TABLE 3. After that, the container was sealed. (In Comparative Example 10, the container was kept open without being sealed.) Then, the container was left for 30 hours at 25°C under sunlight. The solution inside the container was tested for the presence of a decomposition product. The test results are shown in TABLE 3.

**TABLE 3**

| | Glass container (flask) conditions | Oxygen conc. (ppm) inside container | Presence of difluoroacetic acid | Amount (ppm) of difluoroacetic acid generated relative to hemiacetal |
|---|---|---|---|---|
| Example 4 | light shielding | 2000 | not present | n.d. |
| Example 5 | light shielding | 4000 | not present | n.d. |
| Comparative Example 6 | light shielding | 6000 | present | 50 |
| Comparative Example 7 | transparent | 4000 | present | 100 |
| Comparative Example 8 | transparent | 6000 | present | 110 |
| Comparative Example 9 | transparent | 8000 | present | 210 |
| Comparative Example 10 | transparent | 210000 (oxygen conc. in air) | present | 870 |

As shown in TABLE 3, the generation of difluoroacetic acid, which would become a problem in practical use, was not observed in Examples 4 and 5. It is thus apparent in Examples 4 and 5 that there occurred no decomposition of the α,α-difluoroacetaldehyde alkyl hemiacetal. On the other hand, the generation of difluoroacetic acid as a decomposition product was observed in Comparative Examples 6 to 10.

It has been shown by the above results that, in the preservation method of the present invention, it is possible to effectively suppress the generation of difluoroacetic acid over a long term by storing the ifluoroacetaldehyde alkyl hemiacetal under the specific oxygen concentration and light-shielding conditions.

### Industrial Applicability

The preservation method of the present invention is expected to be useful for preservation, storage and distribution of the α,α-difluoroacetaldehyde alkyl hemiacetal as e.g. an intermediate for pharmaceutical and agrichemical products.

## Claims

1. A method for preserving an α,α-difluoroacetaldehyde alkyl hemiacetal of the general formula [3] in a gas-liquid state having gas and liquid phases in a closed container under an atmosphere of oxygen (O₂) or inert gas, 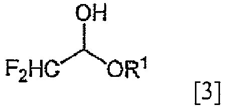 where R¹ represents an alkyl group or a substituted alkyl group,
the method comprising:
controlling the oxygen (O₂) concentration of the gas phase in the container to be 5000 ppm or lower; and then
storing the α,α-difluoroacetaldehyde alkyl hemiacetal in the container under light-shielding conditions.

2. The method according to claim 1,
wherein the storing is carried out in a temperature range of -50°C to 80°C.

3. A method for preserving an α,α-difluoroacetaldehyde alkyl hemiacetal, comprising the following steps:
[First Step] feeding an α,α-difluoroacetaldehyde alkyl hemiacetal of the general formula [3] into a container, thereby forming a gas-liquid state having a gas phase and a liquid phase of the α,α-difluoroacetaldehyde alkyl hemiacetal in the container 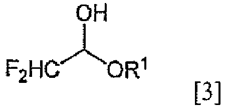 where R¹ represents an alkyl group or a substituted alkyl group;
[Second Step] after the first step, controlling the oxygen concentration of the gas phase in the container to be 5000 ppm or lower by charging oxygen (O₂) or inert gas into the container; and
[Third Step] after the second step, closing the container and then storing the α,α-difluoroacetaldehyde alkyl hemiacetal in the container under light-shielding conditions.

4. The method according to claim 2,
wherein, in the third step, the storing is carried out in a temperature range of -50°C to 80°C.

5. The method according to any one of claims 1 to 4, further comprising:
producing the α,α-difluoroacetaldehyde alkyl hemiacetal of the general formula [3] by reaction of an α,α-difluoroacetate of the general formula [5] with hydrogen (H₂) in an alcohol solvent of the general formula [2] in the presence of a base and a ruthenium catalyst; and using the hemiacetal as a starting raw material 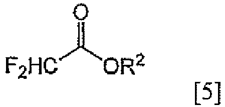
where R² represent an alkyl group or a substituted alkyl group
R¹-OH [2]
where R¹ represents an alkyl group or a substituted alkyl group.

6. The method according to any one of claims 1 to 5,
wherein, in the α,α-difluoroacetaldehyde alkyl hemiacetal of the general formula [3], R¹ is a methyl group or ethyl group.
